# DEMANDE DE BREVET EUROPEEN

(11) **EP 1 867 356 A1**
(43) Date de publication de la demande: **19.12.2007**
(21) Numéro de dépôt: 06115584.2
(22) Date de dépôt: 16.06.2006
(51) Int. Cl.: A61M 5/00, A61M 5/14

(54) **Système pour l'injection de produits de contraste**

(71) Demandeur: Swiss Medical Care, 1004 Lausanne (CH)
(72) Inventeur: Duffour, Hervé, Swiss Medical Care, 1004, Lausanne (CH); Neftel, Frédéric, Swiss Medical Care, 1004, Lausanne (CH)
(74) Mandataire: Grosfillier, Philippe

(57) **Abrégé**

Système pour l'injection de produit de contraste comprenant un mécanisme d'injection, au moins deux réservoirs associés de manière fonctionnelle au mécanisme d'injection, lesdits réservoirs étant adaptés pour contenir respectivement au moins un produit de contraste et un produit de rinçage; et un dispositif de commande, comportant des moyens de programmation, qui est associé de façon fonctionnelle au mécanisme d'injection et susceptible de fonctionner pour programmer de façon sélective une ou plusieurs phases de procédure d'injection; système caractérisé par le fait qu'il comprend un organe déclencheur associé de manière fonctionnelle au dispositif de commande de façon à ce que le dispositif de commande soient adapté pour programmer au moins une phase d'injection de produit de contraste à un débit déterminé suivi d'une phase de rinçage à un autre débit déterminé et une durée programmée pour l'injection du produit de contraste qui est fonction d'un signal donné au cours de l'injection par ledit organe déclencheur lorsque le produit de contraste est visualisé dans une région intérêt.

## Description

### Domaine de l'invention

L'invention concerne l'injection de produits de contraste. Elle se rapporte plus précisément à l'injection de produits de contraste suivi d'une injection d'un produit de rinçage.

### Etat de la technique

On connaît divers systèmes et méthodes permettant d'injecter des produits de contraste. Voir en particulier le brevet américain US 6 385 483 B1 et la demande de brevet américain US 2003/0216643 A1.

### Exposé général de l'invention

Le problème que la présente invention se propose de résoudre réside dans l'usage de produits de contraste au mieux des besoins liés à l'examen, en particulier en réduisant la quantité nécessaire de produit de contraste (et par la même les effets secondaires liés aux produits de contraste) tout en synchronisant l'injection avec la séquence d'acquisition de l'examen proprement dit. Ainsi, l'invention permet de garantir un meilleur succès des procédures d'examen en garantissant que le produit de contraste est bien présent pendant tout le temps nécessaire durant la séquence d'examen, tout en minimisant la quantité injectée.

Dans l'invention, la solution du problème précité consiste en un système pour l'injection de produit de contraste comprenant un mécanisme d'injection, au moins deux réservoirs associés de manière fonctionnelle au mécanisme d'injection, lesdits réservoirs étant adaptés pour contenir respectivement au moins un produit de contraste et un produit de rinçage; et un dispositif de commande, comportant des moyens de programmation, qui est associé de façon fonctionnelle au mécanisme d'injection et susceptible de fonctionner pour programmer de façon sélective une ou plusieurs phases de procédure d'injection; système caractérisé par le fait qu'il comprend un organe déclencheur associé de manière fonctionnelle au dispositif de commande de façon à ce que le dispositif de commande soit adapté pour programmer au moins une phase d'injection de produit de contraste à un débit déterminé suivi d'une phase de rinçage à un autre débit déterminé et une durée non-déterminée pour l'injection du produit de contraste qui est fonction d'un signal donné au cours de l'injection par ledit organe déclencheur lorsque le produit de contraste est visualisé dans une région d'intérêt.

Selon un mode de réalisation de l'invention le système comprend des moyens pour permettre à un utilisateur de programmer la durée d'acquisition des images et/ou la durée pendant laquelle du produit de contraste doit être présent dans la zone d'acquisition des images.

Alternativement, la durée d'acquisition peut être déterminée automatiquement, sur la base de certaines données de bases fournies au système.

Il convient de noter qu'en général, la zone d'acquisition des images ne correspond pas à la zone d'intérêt, cette dernière étant située en amont de la zone d'acquisition des images.

Par "amont", respectivement "aval", on entend la région qui vient avant, respectivement après, la région concernée qui est traversée par le liquide (produit de contraste ou de rinçage) dans le système vasculaire du patient.

Il convient également de relever que la zone d'acquisition d'images peut être statique ou dynamique. Le plus souvent il s'agit d'une zone dynamique correspondant à une zone balayée par l'appareil d'imagerie ou cours de la séquence d'acquisition des images. Dans ce cas de zone d'acquisition dynamique, la zone de présence du produit de contraste est également dynamique car elle doit suivre la zone d'acquisition.

Selon un autre mode de réalisation de l'invention, le système comprend des moyens de traitement qui, pendant la durée d'acquisition d'images, intègrent la durée éventuellement nécessaire au repositionnement de l'appareil d'imagerie avant le début des acquisitions d'images.

L'organe déclencheur est activable manuellement et/ou automatiquement.

Si l'activation est automatique, elle peut l'être par un dispositif d'imagerie tel que l'ordinateur central du scanner CT.

Avantageusement, le système comprend des moyens de sécurité qui sont adaptés pour limiter un volume maximal d'injection de produit de contraste au cours d'une phase. Ce volume maximal peut être programmé pour chaque patient ou être calculé automatiquement en fonction des autres paramètres programmés. Ce volume est notamment utile dans le cas ou le signal serait déclenché manuellement de façon éronnée (trop tardivement ou pas du tout).

L'invention concerne également un procédé qui utilise le système tel que défini précédemment. Le procédé est caractérisé par le fait qu' il comprend au moins une phase d'injection de produit de contraste à un débit déterminé suivi d'une phase de rinçage à un autre débit déterminé, la durée de l'injection du produit de contraste étant fonction d'un signal donné au cours de l'injection par l'organe déclencheur lorsque le produit de contraste est visualisé dans une région d'intérêt.

De préférence, l'activation de l'organe déclencheur initie également un processus d'acquisition d'images.

Selon un mode de réalisation de l'invention, la durée d'injection du produit de contraste est supérieure à la durée nécessaire à la visualisation dans la zone d'observation, ladite durée d'injection étant calculée en fonction de la durée d'acquisition des images.

Selon une variante, on tient compte du volume de produit de contraste situé dans le système vasculaire du patient (entre le point d'injection et la zone d'intérêt) par analyse du temps nécessaire à l'apparition du produit de contraste dans la zone d'intérêt depuis le début de l'injection, afin de minimiser le volume de produit de contraste injecté au patient par l'utilisation d'au moins une partie de ce volume de produit de contraste tampon situé dans le système vasculaire qui sera poussé par le produit de rinçage dont la séquence d'injection sera avancée avant la fin de la séquence d'acquisition tout en permettant d'assurer la présence du produit de contraste dans la zone nécessaire à l'examen pendant toute la durée de la séquence d'acquisition.

Selon un autre mode de réalisation de l'invention, le débit du produit de rinçage est identique à celui du produit de contraste pendant au moins toute la durée restante de la séquence d'acquisition, l'injection du produit de rinçage étant avancée d'un temps au maximum égal à celle défini entre le début de l'injection de produit de contraste et l'instant lors duquel le signal a été déclenché.

Avantageusement, le débit du produit de rinçage est diminué après la fin de la séquence d'acquisition d'images.

Selon une variante, le débit du produit de rinçage est diminué progressivement.

Selon un autre mode de réalisation de l'invention, le volume total de produit de rinçage injecté est programmé initialement.

Selon un autre mode de réalisation de l'invention, le volume total de produit de rinçage injecté est calculé en fonction de paramètres pré-programmés.

L'invention concerne enfin diverses utilisations du système et du procédé tels que définis plus haut.

Selon un mode de réalisation, on active l'organe déclencheur au moment où le produit de contraste pénètre dans la zone d'intérêt.

Cette activation peut être effectuée par un utilisateur, p.ex. un technicien, qui visualise lui-même sur un écran l'arrivée du produit de contraste dans la zone d'intérêt.

Alternativement, l'organe déclencheur est activé automatiquement, consécutivement à un signal transmis par le dispositif d'imagerie lorsque le produit de contraste est détecté dans la zone d'intérêt.

Un domaine d'utilisation particulièrement intéressant est l'imagerie par Scanner CT.

L'invention peut notamment être avantageusement utilisée dans des procédures d'imagerie CT angiographique (CTA). Selon un tel mode de réalisation de l'invention, la séquence d'acquisition correspond à celle des images du coeur. Dans un tel cas, le signal est donné lorsque le produit de contraste apparaît dans une région située à la sortie du ventricule gauche. Selon un tel mode de réalisation de l'invention, la séquence de rinçage est déterminée de manière à garantir que la concentration de produit de contraste présente dans le coeur droit pendant la phase d'acquisition soit inférieure à un seuil déterminé. De préférence, on minimise la quantité de produit de contraste dans le coeur droit.
Un autre domaine d'utilisation de l'invention est l'imagerie du poumon. Selon un tel mode de réalisation de l'invention, la zone d'intérêt est préférentiellement située dans une zone à la sortie du ventricule droit. Avantageusement, la progression de l'apparition du produit de rinçage dans le poumon est calculé précisément en fonction de la séquence d'acquisition.

La présente invention présente plusieurs avantages, en particulier de réduire la quantité de produit de contraste à utiliser pour l'acquisition d'une image, d'abaisser ainsi la toxicité et de réduire les coûts liés à la production d'une image, tout en garantissant la présence de produit de contraste pendant toute la phase nécessaire de l'acquisition de l'image et en diminuant les effets toxiques liés à la quantité totale de produit de contraste injecté au patient.

### Exposé détaillé de l'invention

L'invention sera mieux comprise ci-après au moyen d'exemples détaillés.

Dans le cas d'une imagerie par Scanner CTA, il est important de visualiser les coronaires du coeur. Pour cela il faut s'assurer que le produit de contraste est bien présent dans les coronaires pendant toute la durée ou l'acquisition des images est faite (typiquement entre 6 et 12 secondes pour un Scanner CT à 64 barrettes). En même temps, il est préférable de s'assurer que la partie droite du coeur ne contienne pas ou peu de produit de contraste, afin de pouvoir visualiser le septum et calculer les fractions d'éjections cardiaques.

Ainsi, il est courant aujourd'hui de programmer des volumes de produit de contraste pour un adulte de l'ordre de 70 à 80 ml à un débit de 3 à 5 ml/s, suivi d'une séquence de rinçage à un même débit pour un volume de l'ordre de 30 à 50ml. Ces données permettent de garantir le succès de l'examen quel que soit la fréquence cardiaque, le volume circulatoire et la durée d'acquisition à partir de la présence d'un signal en sortie du ventricule gauche (typiquement au niveau de la crosse aortique), sans optimiser la quantité de produit de contraste injecté, c'est-à-dire avec un risque pour le patient lié aux effets secondaires directement proportionnels au volume de produit de contraste injecté.

Dans le cadre de l'invention, on limite de préférence la dose de produit de contraste (passant typiquement de 70-80 ml à 40-60 ml) en ne programmant pas le volume injecté mais plutôt la durée pendant laquelle le produit de contraste doit être présent dans le coeur gauche (en général la durée pendant laquelle se fait l'acquisition des images). Cette durée doit, éventuellement, tenir compte du temps de repositionnement du scanner (environ 1 à 3 secondes) à partir du moment ou le signal est objectivé à la sortie du coeur gauche. Ainsi l'injection de produit de contraste se fera pendant toute la durée nécessaire à performer un examen correct, évitant ainsi les séquences d'injection insuffisantes (pour lesquelles l'examen risque de devoir être répété) et les quantités de produit de contraste injectées trop importante. En outre il est important de s'assurer d'une vidange de produit de contraste du coeur droit pendant cette phase d'acquisition, ce qui est obtenu par l'injection de produit de rinçage suffisamment rapidement après l'apparition de produit de contraste dans la zone d'intérêt (en général le temps de repositionnement du scanner est supérieur au temps nécessaire au produit de rinçage pour arriver au coeur droit, permettant ainsi d'initier la séquence de rinçage en fonction de l'arrivée de produit de contraste en sortie du coeur gauche tout en tenant compte du temps qu'il aura fallut au produit de contraste pour arriver au coeur gauche depuis le point d'injection pour s'assurer que du produit de contraste est présent dans le coeur gauche tout au long de la séquence d'acquisition). Idéalement, la séquence d'injection du produit de rinçage doit être calculée pour minimiser la quantité totale de produit de contraste injectée au patient, tout en assurant que du produit de contraste est toujours présent dans le coeur gauche pendant toute la durée d'acquisition des images et qu'un minimum de produit de contraste est présent dans le coeur droit pendant cette même période d'acquisition des images.

En procédant ainsi, il est possible d'initier la séquence de rinçage au moment opportun, tout en minimisant l'injection de produit de contraste dont la durée d'injection ne dépassera en général pas la fin de la séquence d'acquisition des images. En outre le débit d'injection de produit de contraste, durant toute la durée de l'injection, restera de préférence fixe, tandis que le débit d'injection du produit de rinçage, après la fin de la séquence d'injection de produit de contraste, pourra éventuellement diminuer. De préférence, cependant, le débit du produit de rinçage reste fixe pendant au moins toute la durée d'acquisition des images. Ces débits d'injection et de produit de contraste peuvent, toutefois, éventuellement varier, pendant chacune des phases. Les séquences d'injections de produit de contraste et de rinçage sont, de préférence, toujours séquentielles, aucune dilution entre le produit de contraste et la solution de rinçage n'étant en principe nécessaire.

## Revendications

1. Système pour l'injection de produit de contraste comprenant un mécanisme d'injection, au moins deux réservoirs associés de manière fonctionnelle au mécanisme d'injection, lesdits réservoirs étant adaptés pour contenir respectivement au moins un produit de contraste et un produit de rinçage; et un dispositif de commande, comportant des moyens de programmation, qui est associé de façon fonctionnelle au mécanisme d'injection et susceptible de fonctionner pour programmer de façon sélective une ou plusieurs phases de procédure d'injection; système **caractérisé par le fait qu'**il comprend un organe déclencheur associé de manière fonctionnelle au dispositif de commande de façon à ce que le dispositif de commande soit adapté pour programmer au moins une phase d'injection de produit de contraste à un débit déterminé suivi d'une phase de rinçage à un autre débit déterminé et une durée non-déterminée pour l'injection du produit de contraste qui est fonction d'un signal donné au cours de l'injection par ledit organe déclencheur lorsque le produit de contraste est visualisé dans une région d'intérêt.

2. Système selon la revendication 1 comportant des moyens pour permettre à un utilisateur de programmer une durée d'acquisition des images à partir de laquelle est calculée la durée d'injection du produit de contraste à partir du signal donné au cours de l'injection.

3. Système selon la revendication 2 comprenant des moyens de traitement qui, dans la durée d'acquisition d'images, intègrent la durée éventuellement nécessaire au repositionnement de l'appareil d'imagerie avant le début des acquisitions d'images pour calculer la durée d'injection.

4. Système selon l'une quelconque des revendications précédentes où l'organe déclencheur est adapté pour être activé manuellement.

5. Système selon l'une quelconque des revendications précédentes où l'organe déclencheur est adapté pour être activé automatiquement.

6. Système selon la revendication 5 **caractérisé par le fait qu'**il est activable directement par un dispositif d'imagerie.

7. Système selon l'une quelconque des revendications précédentes comprenant des moyens de sécurité qui sont adaptés pour limiter un volume maximal d'injection de produit de contraste au cours d'une phase, ledit volume maximal étant de préférence défini en fonction du patient.

8. Procédé utilisant un système tel que défini dans l'une quelconque des revendications précédentes pour l'injection de produits de contraste et l'acquisition d'images, ledit procédé étant **caractérisé par le fait qu'** il comprend au moins une phase d'injection de produit de contraste à un débit déterminé, sans que ni le volume ni la durée ne soient déterminés à l'avance, suivi d'une phase de rinçage à un autre débit déterminé, la durée de l'injection du produit de contraste étant fonction d'un signal donné au cours de l'injection par l'organe déclencheur lorsque le produit de contraste est visualisé dans une région d'intérêt, ceci afin de permettre d'acquérir des images contrastées dans une zone dite "zone d'acquisition d'images".

9. Procédé selon la revendication **caractérisé par le fait que** l'initiation de l'organe déclencheur initie également un processus d'acquisition d'images, ladite initiation pouvant être réalisée manuellement ou automatiquement.

10. Procédé selon la revendication 8 ou 9 dans lequel la durée d'injection du produit de contraste à partir du signal déclencheur est fonction de la durée nécessaire à la présence de produit de contraste dans ladite zone d'acquisition d'images, ladite durée d'injection étant calculée en fonction de la durée d'acquisition des images.

11. Procédé selon la revendication 10 dans lequel on tient compte du volume de produit de contraste tampon situé dans le système vasculaire du patient (entre le point d'injection et la zone d'intérêt), ce volume étant déduit par analyse du temps nécessaire à l'apparition du produit de contraste dans la zone d'intérêt depuis le début de l'injection, afin de minimiser le volume de produit de contraste injecté au patient du fait de l'utilisation d'au moins une partie de ce volume de produit de contraste tampon qui sera poussé par le produit de rinçage avant la fin de la séquence d'acquisition de telle façon que du produit de contraste soit présent dans la zone nécessaire à l'examen pendant toute la durée de la séquence d'acquisition.

12. Procédé selon la revendication 11 dans lequel on injecte le produit de rinçage au plus tôt Tr secondes avant la fin de la séquence d'acquisition, Tr représentant la durée entre le démarrage de l'injection de produit de contraste et le signal, afin de s'assurer qu'il n'y a pas de produit de rinçage dans la zone située en aval de la zone d'intérêt pendant la durée de l'acquisition.

13. Procédé selon la revendication 11 ou 12 dans lequel on injecte suffisamment tôt le produit de rinçage après le déclenchement du signal afin de s'assurer qu'un certain territoire situé en amont de la zone d'intérêt contienne un niveau faible de produit de contraste, mais suffisamment tard pour assurer que la partie de la zone d'acquisition, qui est située en aval de la zone d'acquisition d'images, contienne un niveau suffisamment élevé de produit de contraste pendant la durée de l'acquisition.

14. Procédé selon l'une quelconque des revendications précédentes 10 à 13 dans lequel le débit du produit de rinçage est identique à celui du produit de contraste pendant au moins toute la durée restante de la séquence d'acquisition, l'injection du produit de rinçage étant avancée d'un temps au maximum égal à celui défini entre le début de l'injection de produit de contraste et l'instant lors duquel le signal a été déclenché.

15. Procédé selon l'une quelconque des revendications précédentes 8 à 14 dans lequel le débit du produit de rinçage est diminué après la fin de la séquence d'acquisition d'images.

16. Procédé selon la revendication 15 dans lequel la diminution est progressive.

17. Procédé selon l'une quelconque des revendications précédentes 8 à 16 dans lequel le volume total de produit de rinçage injecté est programmé initialement.

18. Procédé selon l'une quelconque des revendications précédentes 8 à 16 dans lequel le volume total de produit de rinçage injecté est calculé en fonction de paramètres pré-programmés.

19. Utilisation d'un système selon l'une quelconque des revendications 1 à 7 et d'un procédé selon l'une quelconque des revendications 8 à 18 **caractérisée par le fait que** l'on active manuellement ou automatiquement l'organe déclencheur au moment où le produit de contraste est visualisé dans la zone d'intérêt.

20. Utilisation d'un système selon l'une quelconque des revendications 1 à 7 et d'un procédé selon l'une quelconque des revendications 8 à 18 pour l'imagerie par Scanner CT.

21. Utilisation d'un système selon l'une quelconque des revendications 1 à 7 et d'un procédé selon l'une quelconque des revendications 8 à 18 pour les procédures d'imagerie CT Angiographie.

22. Utilisation selon la revendication 21 dans laquelle la séquence d'acquisition correspond à celle des images du coeur.

23. Utilisation selon la revendication 22 dans laquelle le signal est donné lorsque le produit de contraste apparaît dans une région située à la sortie du ventricule gauche.

24. Utilisation selon la revendication 20 ou 23 dans laquelle la séquence de rinçage est déterminée de manière à garantir une certaine concentration de produit de contraste présent dans le coeur droit pendant la phase d'acquisition qui soit inférieure à un seuil déterminé.

25. Utilisation selon la revendication 24 dans laquelle on minimise la quantité de produit de contraste dans le coeur droit.

26. Utilisation d'un système selon l'une quelconque des revendications 1 à 7 et d'un procédé selon l'une quelconque des revendications 8 à 18 pour l'imagerie du poumon.

27. Utilisation selon la revendication 26 dans laquelle la zone d'intérêt est située dans une zone à la sortie du ventricule droit.

28. Utilisation selon la revendication 26 ou 27 dans laquelle la progression de l'apparition du produit de rinçage dans le poumon est calculée précisément en fonction de la séquence d'acquisition.
